# EUROPEAN PATENT SPECIFICATION

(11) **EP 0 644 949 B1**
(45) Date of publication and mention of the grant of the patent: **03.12.1997**
(21) Application number: 93912795.7
(22) Date of filing: 26.05.1993
(51) Int. Cl.: C12P 41/00, C12P 7/40

(54) **A PROCESS FOR THE PREPARATION OF S-(+)-2-(3-BENZOYLPHENYL)PROPIONIC ACID BY ENZYME-CATALYSED ENANTIOSELECTIVE HYDROLYSIS**
VERFAHREN ZUR HERSTELLUNG VON S-(1)-2-(3-benzoylphenyl)PROPIONSÄURE DURCH ENZYMKATALINIERTE ENANTIOSELEKTIVE HYDROLYSE.
PROCEDE DE PREPARATION D'ACIDE S-(+)-2-(3-BENZOYLPHENYL)PROPIONIQUE PAR HYDROLYSE ENANTIOSELECTIVE A CATALYSE ENZYMATIQUE

(30) Priority: 08.06.1992 ES 9201189
(43) Date of publication of application: 29.03.1995
(73) Proprietor: LABORATORIOS MENARINI S.A., 08912 Badalona (ES)
(72) Inventor: CARGANICO, Germano, E-08912 Badalona (ES); MAULEON CASELLAS, David, E-08912 Badalona (ES); PALOMER BENET, Albert, E-08912 Badalona (ES)
(74) Representative: Minoja, Fabrizio
(86) International application number: EP9301321
(87) International publication number: WO9325703

(56) References cited:
- EP-A- 0 227 078
- EP-A- 0 239 470
- EP-A- 0 407 033
- WO-A-91/13163

## Description

### FIELD OF THE INVENTION

The present invention relates to a process for the preparation of optically active S-(+)-2-(3-benzoylphenyl)propionic acid (I), by resolution of the racemate through enantioselective hydrolysis of a reactive ester (II) in an organic medium, which hydrolysis is catalysed by an extracellular lipase of microbial origin. The separation of the resulting free acid from the remaining ester is carried out by extraction in basic medium, to leave the S-ester in the organic phase and the R-acid in the aqueous one. Finally, the desired S isomer is obtained either by aqueous hydrolysis of the remaining reactive S-ester, at constant pH, or enzymatically.

### TECHNOLOGICAL BACKGROUND

A number of antiinflammatory agents of the family of the aryl or alkylarylpropionic acids (i.e. naproxen, ibuprofen, flurbiprofen, etc.) are known, having a chiral center in the α carbon and therefore existing in two optically different forms. The higher activity of the S enantiomer compared with that of the R one is also known [Muller N. et al., Fundam. Clin. Pharmacol. 4, 617 (1990)]. This is also the case of ketoprofen (S-(+)-2-(3-benzoylphenyl)propionic acid), the physiological activity of which mainly resides in the S enantiomer [Yamaguchi T. et al., Folia Pharmacol. Japon. 90, 295 (1987)].

The achievement of the chiral S-(+)-2-(3-benzoylphenyl)propionic acid (I) was approached both by enantioselective synthesis [Fadel A., Synlett. 1, 48 (1992), FR 2,659,968 (21.3.1991)] and by resolution of the racemate, and particularly remarkable is the resolution by diastereomeric crystallization of a (-)-1-phenylpropylamine salt, whereby the S-(+) isomer is obtained in a 22% yield after two recrystallizations and in an optical purity higher than 99% [Nohira H. et al. EP 423,467].

Among the enzymatic methods for the optical resolution, only the stereoselective hydrolysis in aqueous medium has been applied to the racemic 2-(3-benzoylphenyl)propionic acid. Sih et al. [Sih C.L. et al., EP 227,078] describe the resolution of this compound by hydrolysis of the methyl ester thereof, catalysed by a purified form of the lipase from Candida cylindracea, with optical purity values higher than 95% and in a 78% yield. In spite of these results, the procedure seems to be poorly economically convenient due to the need to modify-purify the enzyme. On the other hand, Cerbelaud D. et al. [EP 330529 and FR 2626288] describe the resolution by enantioselective hydrolysis of the nitrile and the corresponding amide catalysed by microorganisms such as Corynebacterium I-774 or Brevibacterium R-312, to obtain the acid in an 88% yield and with an optical purity higher than 95%, even though it should be noted the disadvantage that these reactions are carried out at a high dilution (0.05 M).

The ematioselective hydrolysis of ketoprofen or other antiinflammatory agents of this family, when carried out in an apolar organic solvent saturated with water, showed to have remarkable advantages in the separation of the enantiomers of some chiral lipophilic alcohol acetates [Akita H., et al., Chem. Pharm. Bull. 37, 2876 (1989)], due to the high enantioselectivity of the enzyme in this reaction medium, the higher solubility of the substrate, which allows working at high concentrations and an easy separation of the reaction product, a free acid, from the remaining ester by simple alkali extraction.

EP-A-0407-033 and WO-A-91/13163 disclose the enzymatic resolution of arylpropionic acid, and particularly of Ketoprofen, but the procedure therein diclosed is based on solvent systems different from those described in the present patent application, this latter being characterized by having the possibility of using crude enzyme preparations.

### DISCLOSURE OF THE INVENTION

The present invention relates to a process for the preparation of optically active S-(+)-2-(3-benzoylphenyl)propionic acid (I), 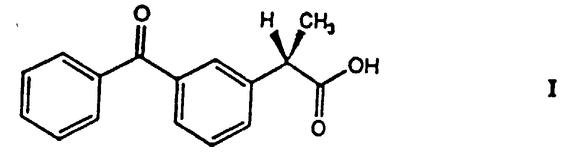 by resolution of the racemate through enantioselective hydrolysis in organic medium of a reactive ester (II), 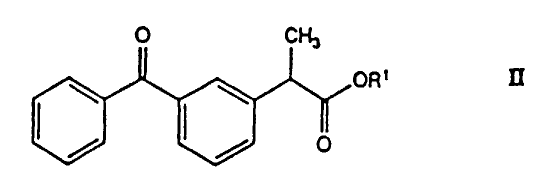 which hydrolysis is catalysed by an extracellular lipase of microbial origin. The separation of the resulting free acid from the remaining ester is carried out by extraction in basic medium, leaving the S-ester in the organic phase and the R-acid in the aqueous one. Finally, the desired S isomer is obtained either by aqueous hydrolysis of the remaining reactive S-ester, at constant pH, or enzymatically.

In formula (II), R¹ is a C₁-C₁₀ straight, branched or cyclic alkyl group, substituted by one or more electron-withdrawing groups, such as halogen, nitro, cyano, -OR², -SR², -COR² [wherein R² can be C₁-C₆ alkyl or cycloalkyl], NR³R⁴ [wherein R³ and R⁴ can be H, C₁-C₆ straight, branched or cyclic alkyl or they can be part of a C₄-C₆ cycle together with the nitrogen and optionally with another heteroatom]. The electron-withdrawing group can be at the α- or β- positions of the R¹ group, thus the ester being referred to as "reactive" due to the faster hydrolysis rate observed for these derivatives. Some specific examples of R¹ groups are 2-chloroethyl, 2,2,2,-trichloroethyl, 2-fluoroethyl, 2,2,2-trifluoroethyl, cyanomethyl, 2-nitropropyl, 2-aminoethyl and N,N-dimethyl-2-aminoethyl.

The process consists in subjecting the substrate (II) to the action of an extracellular carboxylesterase (which enzymes are also named "lipases") from Mucor meihei, in an organic medium saturated with water, as described in the scheme below.

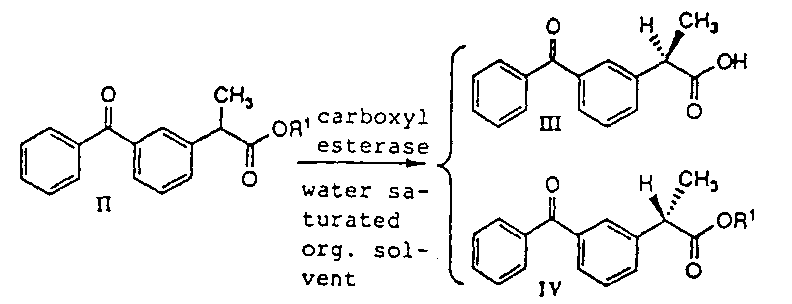

The reaction is carried out in a mixture of two organic solvents, a main water-immiscible one (for example a straight or branched aliphatic ether, or an aliphatic or aromatic hydrocarbon, either halogenated or not) and the other, in a 5 to 25% v/v proportion, which is totally or partially water-miscible, such as a straight or branched aliphatic alcohol, ketone or nitrile or a cyclic ether, typically 1,4-dioxane or tetrahydrofuran, which allows to solubilize some water in the mixture, no more than 5% (v/v).

Among the carboxylesterases capable to catalyse this type of reactions - besides that derived from Mucor meihei commercialized by Amano Pharm. CO. Ltd. (Nagoya, JP) under the commercial name Amano MAP-10 - those derived from microorganisms of the genus, Rhizopus, Aspergillus, Penicillium and Pseudomonas showed a special activity. In all cases, a preference for the R enantiomer was observed, as the R-(-)-2-(3-benzoylphenyl)propionic acid (III) is obtained, whereas the optical antipode thereof, the S isomer, remains in the ester form (IV).

The concentration of the starting ester (II) can be from 0.1 to 1 M, preferably 0.2-0.3 M. The working temperature is from 0 to 60°C, but preferably the reaction is carried out at 20-30°C. The reaction time ranges from 2 to 15 days, preferably 5 to 7 days, and more preferably 6 days. In some cases, the reaction will be carried out until conversions higher than 50%, since, according to the general theory of the irreversible enzyme reactions, [C.S. Chen, Angew. Chem. Int. Ed. Engl., 28, 695 (1989)], and since the interesting compound (S isomer) is the remaining substrate, the optical purity thereof can be optimized only reaching or excessing a 50% conversion.

Once the reaction is over, an alkali is added to allow the separation of the resulting acid (III) from the ester (IV), either by extraction of the latter with a water-immiscible organic solvent (for example ethyl acetate, dichloromethane, diethyl ether or toluene), or by centrifugation or filtration of the resulting suspension, to obtain the remaining S-ester (IV), whereas the acidification of the aqueous phase and the subsequent extraction with an organic solvent give the R-acid (III). The ester (IV) is hydrolysed to S-(+)-2-(3-benzoylphenyl)propionic acid (I) in aqueous media, at controlled pH from 7 to 12, preferably pH 9-10; in this step one of the above mentioned partially water-miscible co-solvents can be added, preferably in a 10 to 60% (v/v) proportion, or a lipase, an esterase or any other hydrolase at their pH optimum can also be used as the catalysts, generally at about neutrality. When the hydrolysis is over, the acid (I) is obtained either by acidification and extraction with a water-immiscible organic solvent as mentioned above, or by centrifugation or filtration of the resulting suspension.

The following example further illustrates the invention.

### Example 1a: Synthesis of the 2-(3-benzoylphenyl)propionic acid chloride (V)

In a 500 ml three-necked flask, 50 g (0.197 mole) of 2-(3-benzoylphenyl)propionic acid are dissolved in 200 ml of dry CCl₄. Subsequently, 50 ml (81.87 g, 0.688 mole) of thionyl chloride dissolved in 100 ml of the same solvent are slowly added. The mixture if refluxed for 5 hours. Solvent is evaporated off under vacuum, 50 ml of CCl₄ are added and the mixture is left to evaporate, repeating the procedure to remove thionyl chloride. 53.2 g of an oily liquid are obtained which is directly used in the subsequent reaction step.

### Example 1b: Synthesis of (±)-2-(3-benzoylphenyl)propionic acid trifluoroethyl ester (II, R¹ = -CH₂CF₃)

In a 1000 ml three-necked flask, 30.19 ml (21.92 g, 0.217 mole) of triethylamine and 15.60 ml (21.67 g, 0.217 mole) of 2,2,2-trifluoroethanol are dissolved in 200 ml of dry dichloromethane. The mixture is stirred on an ice-bath for some minutes and a solution of 53.72 g (0.197 mole) of the acid chloride (V) dissolved in 500 ml of dry dichloromethane is added, keeping temperature below 10°C. The mixture is left under stirring at room temperature for 12 to 24 hours. The crude reaction product is evaporated under reduced pressure. The residue is extracted with 0.1 N NaOH (3x200 ml) and ethyl ether (3x100 ml). The organic phase is washed with 0.1N NaOH (2x50 ml) and dried over sodium sulfate. After that, solvent is removed under vacuum to obtain 66.3 g (97,5% overall yield) of the title ester. 1H N.M.R. (300 MHz, CDCl₃): 7.4-7.9 (m, 9H; Ph-H); 4.35 (m, 2H; CH₂CF₃); 3.88 (dd J=3.7, lH; CHCOO); 1.56 (d J=3, 3H; CH₃).

### Example 1c: Reaction of enantioselective hydrolysis of the ester (II) (R¹ = -CH₂CF₃) catalysed by the lipase from Mucor meihei, Amano M-AP-10

150 g (0.446 mole) of the ester (II) (R¹ = -CH₂CF₃) are dissolved in 1700 ml of a mixture of diisopropyl ether/1,4-dioxane/H₂O (80/20/2.25). Subsequently 223 g of lipase of Mucor meihei (Amano MAP-10^{R}) are added thereto, to obtain a suspension which is kept under strong stirring for 6 days at 27°C. The final mixture is filtered, washing the residue with diethyl ether, extracted with 0.1 N NaOH (3 x 200 ml), dried and the solvent is evaporated off under vacuum to obtain 75.2 g (100% yield) of the trifluoroethyl ester (IV) (R¹ = -CH₂CF₃) of S configuration, in a 98% optical purity. The basic aqueous phase is acidified with 0.5 N HCl to pH 1 and extracted with diethyl ether (3 x 100 ml), dried and the solvent is evaporated under vacuum to obtain 59.4 g of the acid (III) of R-(-) configuration, in an 80% optical purity. Trifluoroethyl ester (IV) (R¹ = -CH₂CF₃): 1H N.M.R. (300 MHz, CDCl₃): 7.4-7.9 (m, 9H; Ph-H); 4.35 (m, 2H; CH₂CF₃); 3.88 (dd J=3.7, 1H; CHCOO); 1.56 (d J=3, 3H; CH3); optical purity = 98%. Acid (III): IR (CHCl₃) 3000-3400, 1730, 1650, 1600, 1460, 1410, 1290, 1200, 1150, 1075 cm-1; [α]²³_{D} = - 44.46° (c 0.98, CHCl₃); optical purity = 80%.

The optical purity of the chiral compounds cited in this and in other embodiments was determined by high performance liquid chromatography (HPLC) by means of a Chiralcel OJ column manufactured by Daicel Chem Ind, Ltd. and a n-hexane/i-propanol/AcOH (80/19.5/0.5) mobile phase.

### Example 1d: Hydrolysis of the trifluoroethyl ester (IV) (R¹ = -CH₂CF₃)

30 g of trifluoroethyl ester (IV) (R¹ = -CH₂CF₃, optical purity = 98%) are dissolved in 2550 ml of a mixture of water and tetrahydrofuran (50% v/v). The mixture is adjusted to pH 9.5 and maintained to this pH for 4 days, by addition of 0.1 N NaOH with a pH-Stat. The volume of the suspension is reduced to 1/5 by evaporation under vacuum, pH is adjusted to 12 by addition of 1 N NaOH and the mixture is extracted with diethyl ether (5 x 100 ml). The aqueous phase is acidified to pH 1 by addition of 1 N HCl, then is extracted with ethyl ether (3 x 10 ml) and dried. After evaporating the solvent under vacuum, 20.4 g (90% yield) of the acid (I) of 93.5% optical purity are obtained. 1H N.M.R. (CDCl₃, 300 MHz) 7.35-7.80 (m, 9H; pH-H); 3.77 (q J=4, 1H; CHCOOH); 1.50 (d J=4, 3H; CH₃); IR (CHCl₃) 3000-3400, 1730, 1650, 1600, 1460, 1410, 1290, 1200, 1150, 1075 cm-1; M.p. = 87-92°C; [α]²³_{D} = + 51.96° (c 1.0, CHCl₃); optical purity = 93.5%.

Alternatively, the hydrolysis is carried out by the action of an enzyme in an aqueous medium. For this purpose, 168 mg (0.5 mmole) of trifluoroethyl ester (IV) (R¹ = -CH₂CF₃) are dissolved in 5 ml of a buffer solution at pH 7.2 and 100 mg of lipase from Mucor meihei (Lipozyme 10000^{R}) are added. The mixture is kept for 6 hours at room temperature. pH is adjusted to 1 by addition of 1 N HCl, the mixture is extracted with ethyl ether (3 x 5 ml), dried and the crude product is analysed by chiral HPLC, showing to contain the acid (I), in an 87% optical purity.

## Claims

1. A process for the preparation of S-(+)-2-(3-benzoylphenyl)propionic acid, according to formula (I), 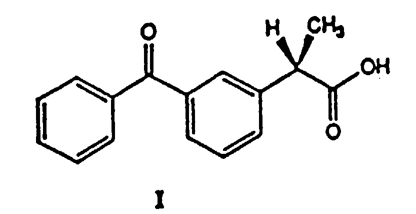 which process comprises the following steps:
a) stereoselective hydrolysis in an organic medium, catalysed by a crude preparartion of lipase from Mucor meihei of the R enantiomer of a racemic reactiv ester of formula (II) 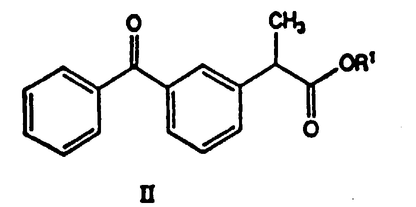 wherein R¹ is a C₁-C₁₀ straight, branched or cyclic alkyl group which is substituted at the α or β positions by one or more electron-withdrawing groups, said hydrolysis being carried out in a mixture of two organic solvents, a main water-immiscible one, and another (in a 5 to 25% v/v proportion) which is totally or partially water-miscible, which solvent allows to dissolve a certain amount of water, no more than 5% (v/v), the concentration of the starting ester (II) being from 0.1 to 1 M, at a temperature from 0 to 60°C for a time from 2 to 15 days;
b) separation of the remaining ester of formula (IV), 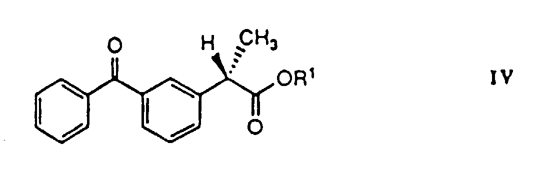 [wherein R¹ have the same meaning as in claim 1a)] of S configuration by extraction in alkaline medium with a water-immiscible organic solvent, or centrifugation or filtration of the resulting suspension;
c) hydrolysis of the ester (IV) to S-(+)-2-(3-benzoylphenyl)propionic acid (I) in aqueous medium, at pH from 7 to 12, in an aqueous pure medium or in the presence of a partially water-miscible co-solvent; or hydrolysis by means of a lipase, an esterase or any other hydrolase as catalysts, at the pH optimum thereof.

2. A process according to claim 1, in which the electron-withdrawing group present in R¹ is halogen, nitro, cyano, -OR², -SR², -COR² [wherein R² can be C₁-C₆ alkyl or cycloalkyl], NR³R⁴ [wherein R³ and R⁴ can be H, C₁-C₆ straight, branched or cyclic alkyl, or they can be part of a C₄-C₆ ring, together with the nitrogen and optionally another heteroatom].

3. A process according to any one of claims 1 or 2, in which the substrate (II) is a reactive ester with R¹ = -CH₂CF₃, -CH₂CH₂F, -CH₂C₂Cl, -CH₂CCl₃, -CH₂CN, -CH(CH₃)NO₂, -CH₂CH₂NH₂ and -CH₂CH₂N(CH₃)₂.

4. A process according to any one of the above claims, in which the R¹ group is -CH₂CF₃.

5. A process according to any one of the above claims, in which the main water-immiscible reaction solvent is straight or branched aliphatic ether, or an aliphatic or aromatic hydrocarbon which can be halogenated or not.

6. A process according to any one of the above claims, in which the main water-immmiscible reaction solvent is a diisopropyl ether.

7. A process according to any one of the above claims, in which the minor reaction solvent is a straight or branched aliphatic alcohol, ketone or nitrile or a cyclic ether.

8. A process according to any one of the above claims, in which the minor reaction solvent is 1,4-dioxane.

9. A process according to any one of the above claims, in which the concentration of the starting ester (II) is 0.2-0.3 M.

10. A process according to any one of the above claims, in which the reaction time is 5-7 days.

11. A process according to any one of the above claims, in which the remaining ester (IV) of S configuration is separated from the acid formed in the reaction by partition between a 0.1 N NaOH aqueous phase and an organic one of diethyl ether.

12. A process according to any one of the above claims, in which the remaining ester (IV), of S configuration, is hydrolysed to S(+)-2-(3-benzoylphenyl)propionic acid (I) in aqueous medium, at pH from 9.0 to 10.

13. A process according to any one of the above claims, in which the hydrolysis of the remaining ester (IV) is carried out in aqueous medium, at pH 9-10, with a partially water-miscible co-solvent in a 10 to 60% (v/v) proportion.

14. A process according to claim 13, in which the co-solvent is tetrahydrofuran in a 50% (v/v) proportion.

## Patentansprüche

1. Verfahren zur Herstellung von S-(+)-2-(3-Benzoylphenyl)propionsäure der Formel (I) 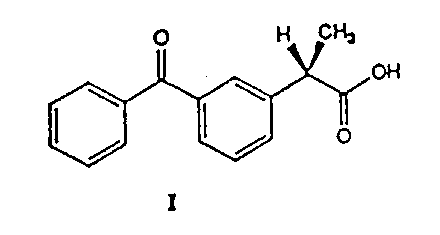 umfassend die folgenden Stufen:
a) stereoselektive Hydrolyse in einem organischen Medium des R-Enantiomeren eines racemischen reaktiven Esters der Formel (II) 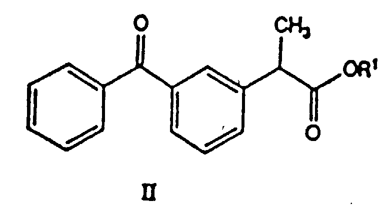 worin R¹ für eine geradkettige, verzweigte oder cyclische C₁-C₁₀-Alkylgruppe steht, die in α- oder β-Position durch ein oder mehrere Elektronen-entziehende Gruppen substituiert ist, die durch eine Rohzubereitung von Lipase von Mucor meihei katalysiert wird, wobei die Hydrolyse in einem Gemisch aus zwei organischen Lösungsmitteln, nämlich einem mit Wasser nicht mischbaren Hauptlösungsmittel und einem anderen Lösungsmittel (in einem Vol./Vol.-Verhältnis von 5 bis 25%), das vollständig oder teilweise wassermischbar ist, wobei dieses Lösungsmittel die Auflösung einer bestimmten Menge von Wasser von nicht mehr als 5% (Vol./Vol.) gestattet, durchgeführt wird, wobei die Konzentration des Ausgangsesters (II) 0,1 bis 1 M beträgt, bei einer Temperatur von 0 bis 60°C über einen Zeitraum von 2 bis 15 Tagen;
b) Abtrennung des zurückbleibenden Esters der Formel (IV) 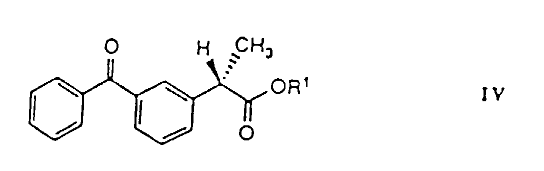 [worin R¹ die gleiche Bedeutung wie in Anspruch 1a) hat] der S-Konfiguration durch Extraktion in einem alkalischen Medium mit einem mit Wasser nicht mischbaren organischen Lösungsmittel oder Zentrifugieren oder Filtrieren der resultierenden Suspension;
c) Hydrolyse des Esters (IV) zu S-(+)-2-(3-Benzoylphenyl)propionsäure (I) in einem wäßrigen Medium bei einem pH-Wert von 7 bis 12, in einem wäßrigen reinen Medium oder in Gegenwart eines teilweise mit Wasser mischbaren Co-Lösungsmittels oder Hydrolyse mittels einer Lipase, einer Esterase oder irgendeiner anderen Hydrolase als Katalysatoren bei ihrem pH-Optimum.

2. Verfahren nach Anspruch 1, dadurch **gekennzeichnet**, daß die in R¹ vorhandene Elektronen-entziehende Gruppe Halogen, Nitro, Cyano, -OR², -SR², -COR² [wobei R² für C₁-C₆-Alkyl oder Cycloalkyl stehen kann], NR³R⁴ [wobei R³ und R⁴ für H, geradkettiges, verzweigtes oder cyclisches C₁-C₆-Alkyl stehen können oder diese einen Teil eines C₄-C₆-Rings zusammen mit dem Stickstoff- und gegebenenfalls einem weiteren Heteroatom sein können] ist.

3. Verfahren nach Anspruch 1 oder 2, dadurch **gekennzeichnet**, daß das Substrat (II) ein reaktiver Ester mit R¹ = -CH₂CF₃, -CH₂CH₂F, -CH₂C₂Cl, -CH₂CCl₃,-CH₂CN, -CH(CH₃)NO₂, -CH₂CH₂NH₂ und -CH₂CH₂N(CH₃)₂ ist.

4. Verfahren nach einem der vorstehenden Ansprüche, dadurch **gekennzeichnet**, daß die Gruppe R¹ -CH₂CF₃ ist.

5. Verfahren nach einem der vorstehenden Ansprüche, dadurch **gekennzeichnet**, daß das mit Wasser nicht mischbare Hauptreaktionslösungsmittel ein geradkettiger oder verzweigter aliphatischer Ether oder ein aliphatischer oder aromatischer Kohlenwasserstoff, der halogeniert sein kann oder nicht, ist.

6. Verfahren nach einem der vorstehenden Ansprüche, dadurch **gekennzeichnet**, daß das mit Wasser nicht mischbare Hauptreaktionslösungsmittel ein Diisopropylether ist.

7. Verfahren nach einem der vorstehenden Ansprüche, dadurch **gekennzeichnet**, daß das Nebenreaktionslösungsmittel ein geradkettiger oder verzweigter aliphatischer Alkohol, Keton oder Nitril oder ein cyclischer Ether ist.

8. Verfahren nach einem der vorstehenden Ansprüche, dadurch **gekennzeichnet**, daß das Nebenreaktionslösungsmittel 1,4-Dioxan ist.

9. Verfahren nach einem der vorstehenden Ansprüche, dadurch **gekennzeichnet**, daß die Konzentration des Ausgangsesters (II) 0,2 bis 0,3 M ist.

10. Verfahren nach einem der vorstehenden Ansprüche, dadurch **gekennzeichnet**, daß die Reaktionszeit 5 bis 7 Tage beträgt.

11. Verfahren nach einem der vorstehenden Ansprüche, dadurch **gekennzeichnet**, daß der zurückgebliebene Ester (IV) der S-Konfiguration von der bei der Reaktion gebildeten Säure durch Aufteilung zwischen einer wäßrigen 0,1 N NaOH-Phase und einer organischen Phase aus Diethylether abgetrennt wird.

12. Verfahren nach einem der vorstehenden Ansprüche, dadurch **gekennzeichnet**, daß der zurückgebliebene Ester (IV) der S-Konfiguration zu S-(+)-2-(3-Benzoylphenyl)propionsäure (I) in einem wäßrigen Medium bei einem pH-Wert von 9,0 bis 10 hydrolysiert wird.

13. Verfahren nach einem der vorstehenden Ansprüche, dadurch **gekennzeichnet**, daß die Hydrolyse des zurückgebliebenen Esters (IV) in einem wäßrigen Medium bei einem pH von 9 bis 10 mit einem teilweise mit Wasser mischbaren Co-Lösungsmittel in einem Verhältnis von 10 bis 60% (Vol./Vol.) durchgeführt wird.

14. Verfahren nach Anspruch 13, dadurch **gekennzeichnet**, daß das Co-Lösungsmittel Tetrahydrofuran in einem Verhältnis von 50% (Vol./Vol.) ist.

## Revendications

1. Un procédé pour la préparation de l'acide S-(+)-2-(3-benzoylphényl)propionique répondant à la formule (I), 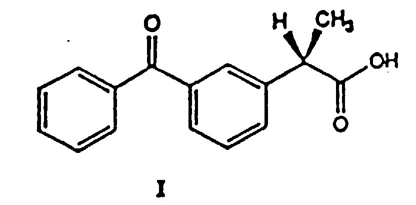 lequel procédé comprend les étapes suivantes :
a) hydrolyse stéréosélective dans un milieu organique, catalysée par une préparation brute de lipase de *Mucor meihei,* de l'énantiomorphe R d'un ester réactif racémique de formule (II) 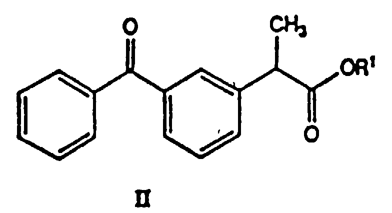 où R¹ est un groupe alkyle linéaire, ramifié ou cyclique en C₁-C₁₀ qui est substitué aux positions α ou β par un ou plusieurs groupes attirant les électrons, ladite hydrolyse étant effectuée dans un mélange de deux solvants organiques, un solvant principal non miscible à l'eau et un autre (dans une proportion de 5 à 25 % en volume) qui est totalement ou partiellement miscible à l'eau, lequel solvant permet de dissoudre une certaine quantité d'eau, non supérieure à 5 % (en volume), la concentration de l'ester de départ (II) étant 0,1 à 1M, à une température de 0 à 60°C pendant un temps de 2 à 15 jours ;
b) séparation de l'ester restant de formule (IV), 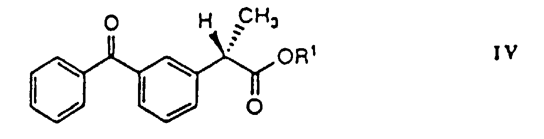 [où R¹ a la même signification que dans la revendication 1a)] de configuration S par extraction dans un milieu alcalin avec un solvant organique non miscible à l'eau, ou centrifugation ou filtration de la suspension résultante ;
c) hydrolyse de l'ester (IV) en acide S-(+)-2-(3-benzoylphényl)propionique (I) dans un milieu aqueux, à pH 7 à 12, dans un milieu aqueux pur ou en présence d'un co-solvant partiellement miscible à l'eau ; ou hydrolyse au moyen d'une lipase, d'une estérase ou de toute autre hydrolase comme catalyseurs, au pH optimal pour celle-ci.

2. Un procédé selon la revendication 1, dans lequel le groupe attirant les électrons présent dans R¹ est un groupe halogéno, nitro, cyano, -OR², -SR², -COR² (où R² peut être un groupe alkyle ou cycloalkyle en C₁-C₆), NR³R⁴ (où R³ et R⁴ peuvent être H, un groupe alkyle linéaire, ramifié ou cyclique en C₁-C₆, ou peuvent faire partie d'un cycle en C₄-C₆ avec l'azote et facultativement un autre hétéroatome).

3. Un procédé selon l'une quelconque des revendications 1 ou 2, dans lequel le substrat (II) est un ester réactif avec R¹ = -CH₂CF₃, -CH₂CH₂F, -CH₂C₂Cl, -CH₂CCl₃, -CH₂CN,-CH(CH₃)NO₂, -CH₂CH₂NH₂ et -CH₂CH₂N(CH₃)₂.

4. Un procédé selon l'une quelconque des revendications ci-dessus, dans lequel le groupe R¹ est -CH₂CF₃.

5. Un procédé selon l'une quelconque des revendications ci-dessus, dans lequel le solvant réactionnel principal non miscible à l'eau est un éther aliphatique linéaire ou ramifié ou un hydrocarbure aliphatique ou aromatique qui peut être halogéné ou non.

6. Un procédé selon l'une quelconque des revendications ci-dessus, dans lequel le solvant réactionnel principal non miscible à l'eau est un éther de diisopropyle.

7. Un procédé selon l'une quelconque des revendications ci-dessus, dans lequel le solvant réactionnel secondaire est un alcool, une cétone ou un nitrile aliphatique linéaire ou ramifié, ou un éther cyclique.

8. Un procédé selon l'une quelconque des revendications ci-dessus, dans lequel le solvant réactionnel secondaire est le 1,4-dioxanne.

9. Un -procédé selon l'une quelconque des revendications ci-dessus, dans lequel la concentration de l'ester de départ (II) est 0,2 à 0,3M.

10. Un procédé selon l'une quelconque des revendications ci-dessus, dans lequel le temps de réaction est de 5 à 7 jours.

11. Un procédé selon l'une quelconque des revendications ci-dessus, dans lequel l'ester restant (IV) de configuration S est séparé de l'acide formé dans la réaction par partage entre une phase aqueuse de NaOH 0,1N et une phase organique d'éther de diéthyle.

12. Un procédé selon l'une quelconque des revendications ci-dessus, dans lequel l'ester restant (IV) de configuration S est hydrolysé en acide S-(+)-2-(3-benzoylphényl)propionique (I) dans un milieu aqueux, à pH 9,0 à 10.

13. Un procédé selon l'une quelconque des revendications ci-dessus, dans lequel l'hydrolyse de l'ester restant (IV) est conduite dans un milieu aqueux, à pH 9 à 10, avec un co-solvant partiellement miscible à l'eau en une proportion de 10 à 60 % (en volume).

14. Un procédé selon la revendication 13, dans lequel le co-solvant est le tétrahydrofuranne en une proportion de 50 % (en volume).
